Europäisches Patentamt

European Patent Office

Office européen des brevets

(11) Publication number: **0 374 451**
**A1**

## EUROPEAN PATENT APPLICATION

(21) Application number: **89120566.8**

(22) Date of filing: **07.11.89**

(51) Int. Cl.⁵: **A61M 5/32**

(30) Priority: **21.12.88 US 287800**

(43) Date of publication of application:
**27.06.90 Bulletin 90/26**

(84) Designated Contracting States:
**AT BE CH DE ES FR GB GR IT LI LU NL SE**

(71) Applicant: **Becton Dickinson and Company**
**One Becton Drive**
**Franklin Lakes New Jersey 07417-1880(US)**

(72) Inventor: **Fayngold, Zevulen**
**52 Edgemere Road**
**Livingston New Jersey(US)**
Inventor: **Zhadanov, Sam**
**Five Sunset Avenue**
**Edison New Jersey(US)**

(74) Representative: **Selting, Günther, Dipl.-Ing. et al**
**Patentanwälte von Kreisler, Selting, Werner**
**Deichmannhaus am Hauptbahnhof**
**D-5000 Köln 1(DE)**

(54) **Entrance gate for a needle disposal shield.**

(57) A gate or cap (12) is provided for slipping over the front end of a needle shield to prevent body fluid in or on a contaminated used needle from flying out the front end of the needle shield either when the shield is being locked in place over the needle after use or during subsequent handling. The cap of the invention includes a front wall of a plurality of flexible flaps (16; 46; 62) which provide, simultaneously, for the required protection while allowing passage of the needle (52; 72) through the cap with ease. The flaps may be in a depressed form to space the front wall or gate from the fingers of a user. There is no wiping of the needle as the needle moves through the cap into position inside the needle shield. No membrane penetration is required. As an additional feature, the gate or cap includes integral spaced annular ridges (14) for frictional gripping of the front end of the shield. It is within the purview of this invention that it may be formed as an integral gate for the front end of a needle shield (44, 66).

FIG. 1

FIG. 2

## AN ENTRANCE GATE FOR A NEEDLE DISPOSAL SHIELD

### Background and Statement of the Invention

This invention relates to blood collection devices. More particularly, this invention relates to protective devices for covering contaminated needles once a blood specimen has been taken in order for the needles to be permanently segregated from contaminating the handler or user of the needle once contamination takes place.

As will be understood by practitioners-in-the-art, a great many needle shield devices have been developed in recent years in the form of axially movable sleeves, which move into place over the contaminated needle from a retracted position over the needle holder prior to use of the needle. These needle shields are designed to lock in place permanently over the needle once the needle has been used, so that the shield cannot be inadvertently moved back over the needle holder exposing the contaminated needle.

Representative of such devices are those taught in U.S. Patent 4,758,231 issued July 19, 1988 which is hereby incorporated by reference in its entirety. The devices taught in that patent were specifically developed for single and multiple sample needle holders for taking blood samples from a patient. Other devices have been developed in the form of axially movable sleeves which move on the barrel of syringes for locking in place once the syringe has been used. Such devices are represented by, for example, U.S. Patent 4,573,976. For both needle holders and syringes, the basic idea is to have a sleeve which is mounted on the barrel of the device so as to expose the needle for use, and then the sleeve is movable axially to lock in place over the needle once it has been used and contaminated. The locking mechanisms are different with a variety of different applications, but they all are arranged to provide a permanent locking of the sleeve over the needle, so that it cannot be exposed for contaminating someone inadvertently after use.

One problem has developed with devices of the kind described above in that there has been incidences of contaminated blood moving out the front end opening of the movable shield, particularly during the movement of the shield to lock in place over the contaminated needle. There is a certain amount of a jerking motion in such a movement for bringing about the permanent locking. Such movements sometimes cause a flinging of contaminated blood from inside the contaminated needle or the outside surface thereof, and over the person who is moving the shield into its locked position. It is to this specific problem that the instant invention is directed.

That is, with this invention, a cap arrangement is provided for placing over the front end opening of the protective needle shield. The cap has a front end gate or net in the form of a plurality of flaps which cover substantially the entire open front end of the needle shield. Thus, once the shield moves into place, the flap arrangement maintains the front end opening substantially closed for any discharge of con taminated blood out the front end of the needle shield.

In addition, because of the specific arrangement of the plurality of flaps and the manner in which they are formed and positioned, there is a greatly reduced incidence of wiping of blood from the external surface of the needle during the movement of the needle shield into place over the needle. That is, during this movement, the needle moves rearwardly through the front end opening of the needle shield.

Prior art devices which provide, for example, puncturable membranes have a tendency to wipe the needle and form beads of blood on the front end opening of the needle shields. With the arrangement according to this invention, such wiping action is substantially obviated. Nevertheless, needles pass freely through the opening of the arrangement herein for insertion and protection inside the movable shield. Moreover, with the preferred arrangement herein the surface of the flaps form a concave front end presentation to the user so that any wiped blood is spaced from the fingers of the user.

Other objects and advantages of this invention will be apparent from the following description, the accompanying drawings and the appended claims.

### Description of the Drawings

Fig. 1 is a sectional view of a front gate or cap for a needle shield and illustrating one embodiment of the invention;

Fig. 2 is a plan view of the front or left-hand end of the cap of Fig. 1;

Fig. 3 is a partial longitudinal sectional view illustrating another embodiment of the invention wherein a portion of the front end of a needle shield has a cap of the invention in the form of an integral front end gate for the shield; and

Fig. 4 is a partial longitudinal sectional view illustrating yet another embodiment of the invention also with a portion of the front end of a needle shield with the cap of the invention in the form of an integral front end gate with the flaps extending outwardly from the front end of the shield.

Detailed Description of the Invention

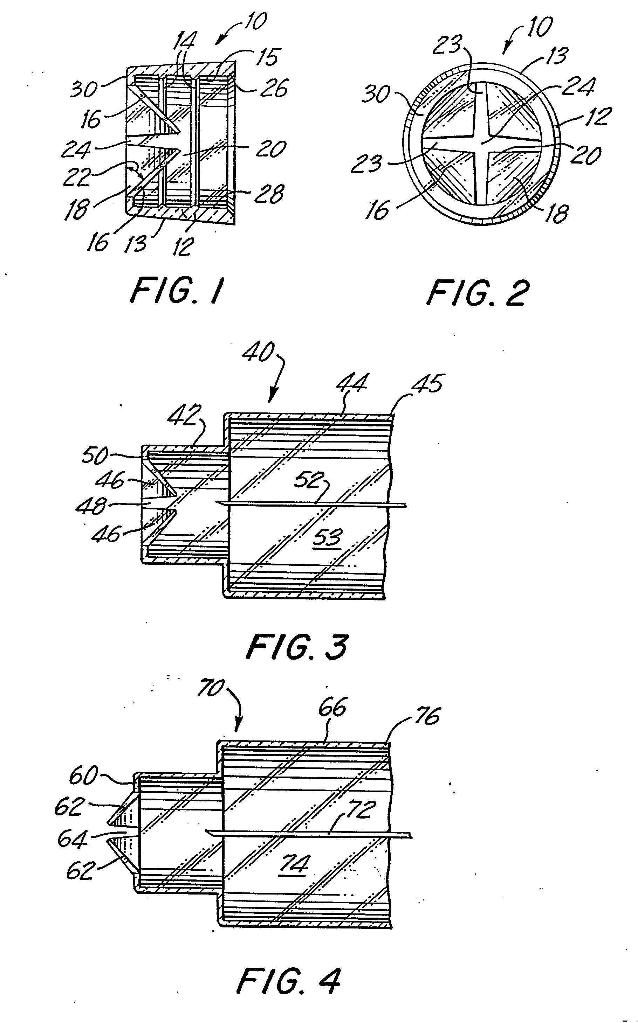

Referring to the drawings in which like reference characters refer to like parts throughout the several views thereof, Fig. 1 illustrates the invention as employed as a gate or cap arrangement for frictionally gripping the front end of a conventional needle shield of the kind discussed in the aforementioned U.S. Patent No. 4,758,231 and used to move into place over a contaminated needle.

In Fig. 1, the device 10 includes a substantially cylindrical body 12. As can be seen in Fig. 1 the outer surface 13 of cap body 12 is tapered slightly in order to ease the process of the molding thereof. Furthermore, the internal surface 15 of cap body 12 includes a plurality of spaced ribs 14 which serve to frictionally grip the outer surface of a needle shield when the cap or gate arrangement 10 is moved over the front end of a needle shield. Ribs 12 may or may not be utilized depending upon other factors for frictionally gripping the needle shield. In this connection, the innermost surface 26 of the internal surface 15 of cap body 12 is chamfered to facilitate the initial introduction of the cap body 12 onto the outer front edge of a needle shield.

As can be seen in Figs. 1 and 2, front surface 30 of cap 12 forms a circumferential flange integral with wall 12 and substantially at right angles thereto. Flange 30 includes a plurality of integral flaps 16 formed by cutting a cross-type division of the front end 30 of cap 12 along the lines 23 shown in Fig. 2. Because of this, a plurality of flaps 16 are formed which are deliberately angled as shown by the angle 22 inwardly to provide a ready passage 24 for a needle passing therethrough. In order to achieve the desired positioning of flaps 16, and depending upon materials being utilized flaps 16 may be thicker at their outermost point 18, and gradually thinner to the innermost point 20 thereof. In the embodiment shown in Fig. 1, the plurality of flaps 16 form a concave surface which is spaced from the fingers of a user, as discussed above.

Representative dimensions, for example, are a thickness of 0.025 inches at point 18 and a thickness of 0.010 inches at point 20. Angle 22, again, as representative only, is about 46.1 degrees from the surface 30 and may be angled within the range of between about 30° and 60°.

Referring now to Fig. 3, a further embodiment of the invention is shown generally at 40 in the form of a front end gate arrangement utilizing the invention as being integral with the front end neck 42 of a needle shield 44. In this arrangement, the front end wall 50 of neck 42 includes integral flaps 46 similar to flaps 16 shown in the Figs. 1 and 2 embodiment. A passage 48 is formed for a needle 52 moving into the internal chamber 53 of needle shield 44 when it is moved into a locking position over a contaminated needle. In this connection, the front end of a conventional needle shield 44 includes an annular neck 42 forming the open front end thereof, similar to the arrangement shown in U.S. Patent 4,758,231 mentioned above. The opposite open end 45 extends an appropriate length not shown for receiving a syringe barrel or needle holder as will be understood by practitioners-in-the-art.

Fig. 4 shows a further embodiment of the invention 70 wherein the front end gate arrangement utilizing the invention is integral with the front end neck 68 of a needle shield 66. In this arrangement, flaps 62 extend outwardly from front end wall 60. Thus, needle 70 moves inwardly through passage 64 into chamber 74 for containment therein. With this device, as with device 40 in the Fig. 3 arrangement, end 76 extends an appropriate length, not shown, for receiving a syringe barrel or needle holder.

While the cap or gate of the invention may be separately configured as shown in Fig. 1 to be placed over the front end of a blood collection needle shield as shown in the Figs. 1 and 2 embodiment, preferably, the cap or gate will be formed as an integral portion of the needle shield arrangement. Preferably, it will be of a transparent or translucent molded thermoplastic such as polyethylene, for example. Other materials which may be used, as will be appreciated by practitioners-in-the-art, include various thermoplastics such as polypropylene, polycarbonate, and polyvinyl chloride, as well as elastomers and metals.

Whereas, as discussed above, specific embodiments of the cap or gate arrangement have been shown, it is to be understood that it is within the purview of this invention to provide other forms of cap flaps for the gating or covering of the front end of a movable needle shield. For example, more divider cuts such as 23 shown in Fig. 2 may be incorporated to provide a larger number of flaps 16, for example. The idea of course is to configure the gating so as to allow movement easily of a contaminated needle through the gate without wiping of the needle so that there is no blood collected on the front end or outer surface of the cap or gate for subsequent contamination. Nevertheless, simultaneously, the gate or cap arrangement of the invention prevents any flinging of blood outwardly through the open end of the movable protective shield once it is locked in place or during the actual locking maneuver.

While the forms of apparatus herein described constitute preferred embodiments of the invention, it is to be understood that the invention is not limited to these precise forms of apparatus, and that changes may be made therein without departing from the scope of the invention which is defined in the appended claims.

**Claims**

1. A cap for forming an entrance gate for a contaminated needle shield, characterized by

(a) a cylindrical body;

(b) said cylindrical body having one open end for receiving the open end of a contaminated needle shield;

(c) frictional gripping means on the internal surface of said cylindrical body for gripping the open end of a contaminated needle shield;

(d) a gated end on said cylindrical body on the end thereof opposite said open end;

(e) said gated end comprising
(1) a circumferential flange positioned substantially at right angles to the wall of said cylindrical body;
(2) said circumferential flange being integral with the wall of said cylindrical body;
(3) a plurality of flaps suspended from and integral with the edge of said circumferential flange opposite said cylindrical body;
(4) each said flap extending toward said open end of said cylindrical body at an angle to said circumferential flange; and

(f) whereby said plurality of flaps prevent passage of contaminated blood outwardly from a contaminated needle shield while allowing nonwiping passage of a contaminated needle therethrough.

2. The cap of Claim 1, further characterized by

(a) four flaps are integral with said circumferential flange.

3. The cap of Claim 1, further characterized by

(a) said angle of each of said plurality of flaps from said circumferential flange is about 46.1 degrees.

4. The cap of Claim 1, further characterized by

(a) each said flap has a decreasing thickness from said circumferential flange to the opposite end thereof.

5. The cap of Claim 1, further characterized by

(a) said frictional gripping means is a plurality of spaced apart annular ribs.

6. A shield assembly for a contaminated needle, characterized by

(a) a cylindrical needle shield body;

(b) said needle shield body having an open end for receiving a syringe barrel or a needle holder;

(c) an integral annular neck positioned on the end of said cylindrical needle shield body opposite said open end;

(d) a gated end on said integral annular neck on the end thereof opposite said needle shield body;

(e) said gated end comprising
(1) a circumferential flange positioned substantially at right angles to the wall of said neck;
(2) said circumferential flange being integral with the wall of said neck;
(3) a plurality of flaps suspended from and integral with the edge of said flange opposite said neck;
(4) each said flap extending toward said cylindrical needle shield body at an angle to said circumferential flange; and

(f) whereby said plurality of flaps prevent passage of contaminated blood outwardly from said contaminated needle shield while allowing nonwiping passage of a contaminated needle therethrough.

7. The assembly of Claim 6, further characterized by

(a) four flaps are integral with said circumferential flange.

8. The assembly of Claim 6, further characterized by

(a) said angle of each of said plurality of flaps from said circumferential flange is about 46.1 degrees.

9. The assembly of Claim 6, further characterized by

(a) each said flap has a decreasing thickness from said circumferential flange to the opposite end thereof.

FIG. 1

FIG. 2

FIG. 3

FIG. 4

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | GB-A-2 114 006 (STERIMATIC HOLDINGS LTD) <br> * Figures 3,5; page 2, lines 125-127 * <br> --- | 1 | A 61 M 5/32 |
| A | DE-U-8 803 216 (TRANSCOJECT GESELLSCHAFT FÜR MEDIZINISCHE Geräte mbH & CO. KG) <br> * Figures 1,2; page 4, lines 13-16 * <br> --- | 1 | |
| A | WO-A-8 702 254 (PHYSIONIC GESELLSCHAFT AIR MEDIZIN UND SYSTEMTECHNIK GmbH) <br> * Figures 15,16; page 16, line 23 - page 17, line 1 * <br> --- | 1 | |
| A | EP-A-0 268 445 (STERIMATIC HOLDINGS LTD.) <br> * Figures 1,2; column 2, lines 23-25 * <br> ----- | 1 | |

TECHNICAL FIELDS SEARCHED (Int. Cl.5)

A 61 M

The present search report has been drawn up for all claims

| Place of search | Date of completion of the search | Examiner |
|---|---|---|
| THE HAGUE | 22-03-1990 | SEDY, R. |